(19) Europäisches Patentamt
European Patent Office
Office européen des brevets

(11) EP 2 094 863 B1

# (12) EUROPEAN PATENT SPECIFICATION

(45) Date of publication and mention of the grant of the patent:
**06.08.2014 Bulletin 2014/32**

(21) Application number: **07851574.9**

(22) Date of filing: **17.12.2007**

(51) Int Cl.:
*C12Q 1/68* (2006.01) *C07K 14/00* (2006.01)

(86) International application number:
**PCT/KR2007/006606**

(87) International publication number:
**WO 2008/075871 (26.06.2008 Gazette 2008/26)**

## (54) PEPTIDE NUCLEIC ACID OLIGOMERS COMPRISING UNIVERSAL BASES, PREPARATION METHODS THEREOF, AND KITS, DEVICES AND METHODS FOR THE ANALYSIS, DETECTION OR MODULATION OF NUCLEIC ACIDS USING THE SAME

PEPTIDNUKLEINSÄURE-OLIGOMERE MIT UNIVERSELLEN BASEN, VERFAHREN ZU IHRER HERSTELLUNG SOWIE KITS, VORRICHTUNGEN UND VERFAHREN ZUR ANALYSE, ERKENNUNG ODER MODULATION VON NUKLEINSÄUREN DAMIT

OLIGOMÈRES D'ACIDES NUCLÉIQUES PEPTIDIQUES COMPRENANT DES BASES UNIVERSELLES, PROCÉDÉS POUR LEUR PRÉPARATION ET TROUSSES, DISPOSITIFS ET MÉTHODES POUR L'ANALYSE, LA DÉTECTION OU LA MODULATION D'ACIDES NUCLÉIQUES AU MOYEN DE CES OLIGOMÈRES

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **18.12.2006 KR 20060129517**

(43) Date of publication of application:
**02.09.2009 Bulletin 2009/36**

(73) Proprietor: **Panagene, Inc.**
**Daejeon 305-804 (KR)**

(72) Inventors:
- **PARK, Hee Kyung**
  **Daejeon 305-358 (KR)**
- **LIM, Jong Chan**
  **Daejeon 305-772 (KR)**
- **HA, Sung Jin**
  **Gyeonggi-do 435-058 (KR)**
- **KIM, Serka**
  **Daejeon 305-345 (KR)**
- **CHOI, Jae Jin**
  **Daejeon 305-335 (KR)**
- **MIN, Jung Hyun**
  **Daejeon 300-120 (KR)**
- **YUN, Jin Won**
  **Daejeon 305-503 (KR)**

(74) Representative: **Hiebl, Inge Elisabeth et al**
**Kraus & Weisert**
**Patent- und Rechtsanwälte**
**Thomas-Wimmer-Ring 15**
**80539 München (DE)**

(56) References cited:
**WO-A2-2006/072368 US-A1- 2005 042 638**

- **FREY K.A. AND WOSKI S.A.: 'Fluoroaromatic universal bases in peptide nucleic acids' CHEMICAL COMMUNICATIONS no. 19, 2002, pages 2206 - 2207, XP008131232**
- **LOAKES D.: 'The applications of universal DNA base analogues' NUCLEIC ACIDS RESEARCH vol. 29, no. 12, 15 June 2001, pages 2437 - 2447, XP002487174**
- **KOHLER O. AND SEITZ O.: 'Thiazole oranges as fluorescent universal base in peptide nucleic acids' CHEMICAL COMMUNICATIONS no. 23, 2003, pages 2938 - 2939, XP008131541**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

[Technical Field]

The present invention relates to peptide nucleic acid (hereinafter, referred to as 'PNA') oligomers comprising universal bases, preparation methods thereof, and kits, devices and methods for the analysis, detection or modulation of nucleic acids using the same. More specifically, it relates to PNA oligomers with remarkably increased specificity upon hybridization with nucleic acids, which comprises at least one universal base incorporated in their base sequences, the base sequences having at least 60% purine bases or at least four contiguous purine bases preparation methods thereof, and kits, devices and methods for the analysis, detection or modulation of nucleic acids using the same.

[Background Art]

Peptide nucleic acids, whose nucleobases are linked by peptide bonds, not by phosphate bonds, have been reported in 1991 for the first time (Nielsen PE, Egholm M, Berg RH, Buchardt O, "Sequence-selective recognition of DNA by strand displacement with a thymine-substituted polyamide", Science 1991, Vol. 254, pp1497-1500). PNAs are not found in nature and made by chemical synthesis. PNAs undergo hybridization reaction with natural nucleic acids having complementary base sequences thereto to form double strands. A PNA/DNA double strand is more stable than a DNA/DNA double strand, and a PNA/RNA double strand is more stable than a DNA/RNA double strand, provided that they have the identical number of nucleobases. The most common backbone of peptide nucleic acid has the structure in which N-(2-aminoethyl) glycines are repeatedly linked by amide bonds. The backbone of PNA is electrically neutral, whereas that of natural nucleic acid is negatively charged.

Four nucleobases in PNA occupy about the same space and have almost the same distance from one another asthose of natural nucleic acids. PNAs are more stable chemically and biologically than natural nucleic acids, because they are not degraded by nucleases or proteases. Moreover, PNA is electrically neutral, and thus, the stability of a PNA/DNA or PNA/RNA double strand is not influenced by salt concentration. Due to these properties, PNAs can recognize complementary base sequence better than natural nucleic acids, so that theycan be applied for diagnosis, and other biological or medical purposes. For example, when PNA binds with its complementary DNA/RNA in a cell, it can act as antisense or antigene, which inhibits physiological functions of the DNA or RNA, thereby to modulate functions of nucleic acid.

In case that a base sequence is identified or detected in a homogeneous solution with a probe of known base sequence, typically one sequence is identified at one time. It is difficult to detect more than a few sequences simultaneously by using fluorescent dyes of different colors. In compassion, in case that a probe immobilized on the surface of a solid support is used, much larger number of base sequences can be detectedsimultaneously with much larger number of probes. DNA microarrays on which several hundred thousands of probes are arrayed in two-dimension have been already commercialized. PNA microarrays or PNA chips employing PNA probes instead of DNA probes have also been known (Brandt O, Hoheisel JD, "Peptide nucleic acids on microarrays and other biosensors," Trends Biotechnology 2004, Vol. 22, pp617-622). A method has been known to make simultaneous detection of target nucleic acids with a large number of probes by immobilizing PNA probes on distinguishable,several micrometer-sized microbeads or microspheres (Rockenbauer E, Petersen KH, Vogel U, Bolund L, Kølvraa S, Nielsen KV, Nexø BA, "SNP genotyping using microsphere-linked PNA and flow cytometric detection", Cytometry Part A 2005, Vol. 64A, pp80-86). Fluorescence is widely used to detect whether or not complementary base sequence is hybridized to a probe, but electric methods have also been known to detect nucleic acids by using a field-effect transistor with PNA immobilized on silicon semiconductor or silicon nanowire [F. Uslu et al. "Labelfree fully electronic nucleic acid detection system based on a field-effect transistor device", Biosensors and Bioelectronics 2004, Vol. 19, pp 1723-1731; J. Hahm and C. M. Lieber, "Direct ultrasensitive electrical detection of DNA and DNA sequence variations using nanowire nanosensors", Nano Letters 2004, Vol. 4, pp 51-54]. A device to detect base sequence by measuring thechange of impedance before and after hybridization of target nucleic acids with PNA probes has also been reported [A. Macanovic et al. "Impedance-based detection of DNA sequences using a silicon transducer with PNA as the probe layer", Nucleic Acids Research 2004, Vol. 32, e20].

Since a probe gains massafter hybridization with a target nucleic acid, a base sequence can be detected from mechanical change resulted therefrom. The frequencies of a microcantilever or a surface acoustic wave (SAW) sensor are different before and after DNA or RNA is bound thereto, so it can be used for detection. Microcantilevers and SAW sensors with PNA have been reported [S. Manalis and T. Burg, US Patent 7,282,329 "Suspended microchannel detectors" P. Warthoe and S. Iben, US Patent Application Publication 2004/0072208 A1 "Surface acoustic wave sensors and method for detecting target analytes"].

PNA probes have limitationsto be designed because of their electric neutrality, differently from DNA probes. PNA probes comprising contiguous purine bases (adenine, guanine), particularly guanines, may strongly bind with DNA in a non-specific manner (Jan Weiler et al., "Hybridisation based DNA screening on peptide nucleic acid (PNA) oligomer

arrays", Nucleic Acids Research, 1997, vol. 25, No. 14, pp2792-2799). PNA probes are also known to bind non-specifically with DNA in case that PNA oligomers self-aggregate due to their low solubility in water (A. J. Tackett et al., "Non-Watson-Crick interactions between PNA and DNA inhibit the ATPase activity of bacteriophase T4 Dda helicase" Nucleic Acids Research 2002 Vol. 30 pp950-957). The low solubility of PNA oligomers in water are problematic especially when their purine base content exceeds 60% out of the total bases thereof. Thus, if a PNA probe complementary to a specific target sequence should be used to detect polymorphism or mutation and such a PNA probe has a base sequence comprising contiguous purine bases or high (60% or more) purine content, it may not detect the target sequence specifically. For example, in case of detecting a point mutation in a target sequence wherein purine bases are contiguously present on one side of the mutation point, while pyrimidine bases are contiguously present on the other side the mutation point, it is not possible to construct a PNA probe that comprises of only natural bases and does not have contiguous purine bases.

On the basis of outcome of researches to reveal genetic information of organisms, such as genome project, base sequences associated with diseases, such as single nucleotide polymorphisms (SNPs), mutations, pathogenic bacteria or viruses and the like, have been found. In addition, methods have been very rapidly developed to analyze the genetic information. Among them, technologies using DNA oligomers have been employed in various fields, including as primers for polymerase chain reaction (PCR), primers for real-time PCR, probes for Northern blot or Southern blot, probes for microarray or fluorescence *in situ* Hybridization (FISH), probes for MALDI-TOF mass spectrometry, and tags for Serial Analysis of Gene Expression (SAGE). These technologies are based on the specific hybridization of primers or probes having complementary base sequences to target nucleic acids to be detected, when the target nucleic acids have known genetic information. In case that genetic information is derived from amino acid sequence, or diverse genetic variations occur, there may be a region that various bases may be present instead a specific base. Such a region is called a polymorphic region. If a polymorphic region has a variation of all four bases (A, G, T, C), 4 kinds of target-specific oligomers must be manufactured. For a polymorphic region with two such variation points, 16 kinds of oligomers must be manufactured. In order to simplify thisproblem, a universal base has been designed. This artificial base can hybridizewith two or more types of bases, differently from natural nucleobases such as adenine, thymine, guanine, cytosine and uracil. When a probe or a primer is designed for a domain containing a polymorphic region, a universal base is substituted for a natural nucleobase, thereby one kind of probe or primer can detect base sequences having various nucleobases in the complementary position to that of the universal base. Due to such advantages, various universal bases useful as probes or the like have been developed. For example, T. A. Millican et al. (Nucleic Acids Research, 1984, 12, 7435-7453) have designed 1,2-dideoxy-D-and 1,2-dideoxy-1-phenyl-β-D-ribofuranose by simply eliminating base or by replacing base with phenyl group, so that they do not undergo specific hybridization by hydrogen bond. These bases can be coupled with all four types of bases, though they have lower binding strength than natural bases. Thereafter, hypoxanthine, xanthine and deaminated guanine (R. Eritja et al., Nucleic Acids Research, 1986, 14, 8135-8153), and 2'-deoxyinosine represented by Formula (1) (Frank Seela and Klaus Kaiser, Nucleic Acids Research, 1986, 14, 1825-1844), which can bind with all four types of bases, as well as 5'-fluorodeoxyuridine (J. F. Habener et al., 1988, Proceedings of the National Acaddemy of Sciences, 85, 1735-1739), methoxycytosine and 6H,8H-3,4-dihydro-pyrimido[4,5-c][1,2]oxazin-7-one (P. K. Thoo et al., 1989, Nuclic Acids Research, 17, 10373-10383), which can bind with two types of bases (adenine and guanine), have been developed.

[Formula 1]

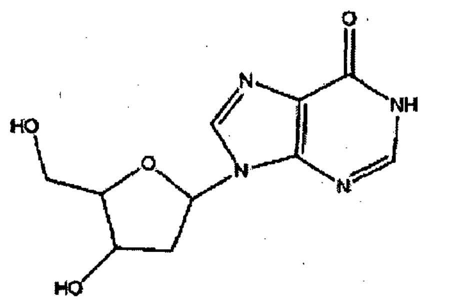

Recently, universal bases which can undergo hybridization by hydrogen bond with all four types of bases, such as 3-nitropyrrole (D. E. Bergstrom et al., 1995, Journal of American Chemical Society, 117, 1201-1209) of Formula (2) and 5-nitroindole of Formula (3) (D. Loakes et al., 1995, Nucl. Acids Res., 23, 2361-2366), have been developed. Since these bases hybridize to all types of natural bases with about the same strength, they have reduced variability in reactivity depending on the types of bases, and so are widely used.

[Formula 2]

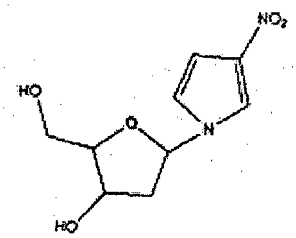

[Formula 3]

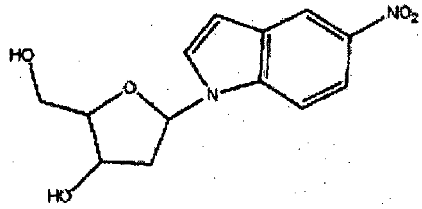

The literature, D. Laokes, "Survey and Summary: The applications of universal DNA base analogues", Nucleic Acids Research, 2001, vol. 29 pp2437-2447, has exemplified universal bases represented by the following Formulas:

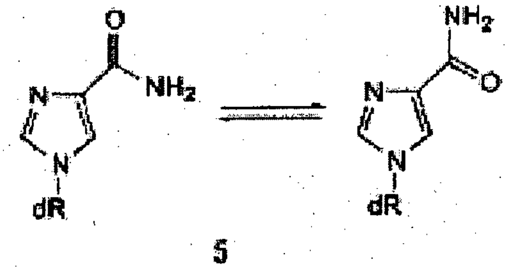

5

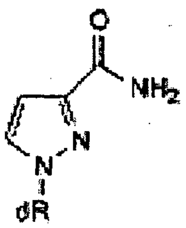

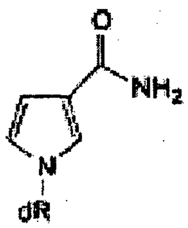

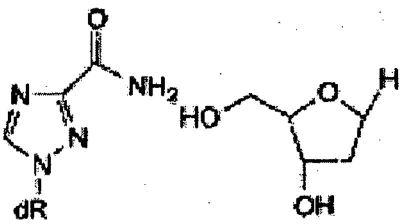

6 7 8 9

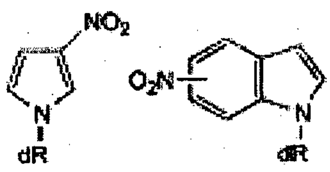

**10**

**11 (5-nitro)**
**12 (4-nitro)**
**13 (6-nitro)**

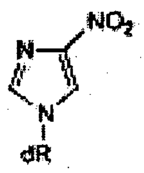

**14**

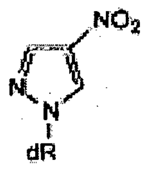

**15**

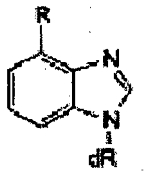

**16**, R = $NO_2$
**17**, R = $NH_2$

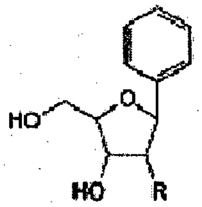

**20a**, R = OH
**20b**, R = H

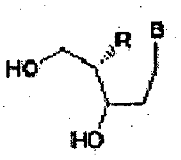

Acyclic sugars

B =

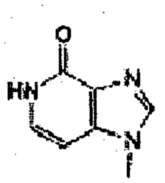

**18**, R = H
**21**, R = OMe

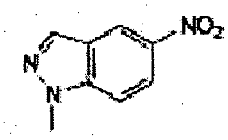

**19**, R = OMe

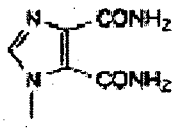

**22**, R = OMe

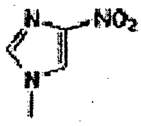

**23**, R = OMe

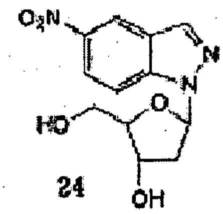

**24**

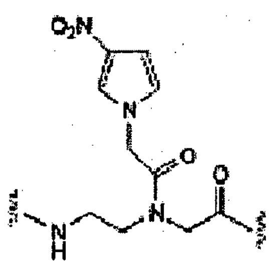

**25**

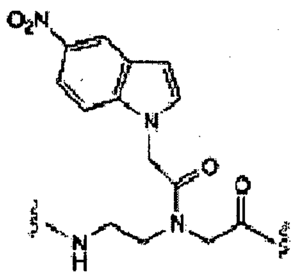

**26**

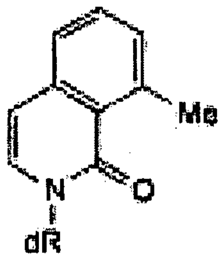
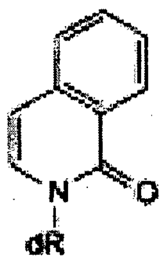

37 38

A few examples of PNA to which universalbases are incorporated have been known (Zhang et al., "Peptide Nucleic Acid-DNA Duplexes Containing the Universal Base 3-Nitropyrrole", METHODS, 2001, vol. 23, pp132-140; Challa et al., "Nitroazole Universal Bases in Peptide Nucleic Acids", Organic Letters, 1999, vol. 1, pp1639-1641). However, they have primarily concerned stability of PNA-DNA bonds in case of incorporating universal bases, but have no mention on PNA sequence containing contiguous purine bases or high content of purine bases.

[Disclosure]

[Technical Problem]

In order to solve the problems of prior arts as described above, the present inventors manufactured PNA oligomers comprising universal bases, by incorporating at least one universal base to PNAs having at least 60%purine bases or at least four contiguous purine basesin their base sequences, and found that PNA microarrays, PNA chips and the like comprising the PNA oligomers as probes can detect nucleic acids containing single nucleotide polymorphisms, mutations or the like, with high specificity (ratio of perfect match signal to single mismatch signal), and completed the present invention. Thus, the first object of the invention is to provide a PNA oligomer comprising at least one universal base.
The second object of the invention is to provide a probe comprising the PNA oligomer.
The third object of the invention is to provide a process for preparing the PNA oligomer.
The fourth object of the invention is to provide a kit containing the PNA oligomer for analyzing, detecting or modulating nucleic acids.
The fifth object of the invention is to provide a device for analyzing or detecting nucleic acids by using the PNA oligomer.
The sixth object of the invention is to provide a method of analyzing, detecting or modulating nucleic acids by using the PNA oligomer.

[Technical Solution]

The first aspect of the present invention relates to a PNA oligomer with increased solubility in water and specificity upon hybridization with nucleic acid, which has at least one universal base, capable of forming a base pair with natural DNA or RNA bases, incorporated in its base sequence, the base sequence containing at least 60% purine bases, or at least four contiguous purine bases and which consists of the base sequence represented by General Formula (1):

[General Formula 1]

$$A_p \; B_q - C_r$$

, wherein $A_p$ and $C_r$ each represent a base sequence complementary to a base sequence other than a specific base sequence, e.g. in polymorphic or mutated region, of a target nucleic acid;
Bqrepresents a base sequence complementary to a specific base sequence, e.g. in polymorphic or mutated region, of a target nucleic acid;
wherein the target nucleic acid comprises the specific base sequence that contains at least 60% pyrimidine bases or at least four contiguous pyrimidine bases
p, q and r are the number of bases, p and r each represent an integer from 0 to 15, and q is an integer from 1 to 30, provided that p+q+r is an integer from 10 to 30; and

$A_p$-$B_q$-$C_r$ is a base sequence to which one or more universal bases capable of forming a base pair with natural DNA or RNA bases have been incorporated.

The second aspect of the invention relates to a probe comprising said PNA oligomer.

The third aspect of the invention relates to a process **for preparing PNA oligomer as defined above with increased solubility in water and specificity upon hybridization with nucleic acid, which comprises the steps of:**

1) examining whether the PNA oligomer to be designed comprises at least 60% purine bases, or at least four contiguous purine bases, in its base sequence;
2) synthesizing PNA monomers comprising natural bases and universal bases, respectively;
3) incorporating at least one universal base capable of forming a base pair with natural DNA or RNA bases to the base sequence with said PNA monomer, to prepare the PNA oligomer.

The fourth aspect of the invention relates to a kit comprising said PNA oligomer for analyzing, detecting or modulating the nucleic acid.

The fifth aspect of the invention relates to a device for analyzing or detecting nucleic acids by using said PNA oligomer.

The sixth aspect of the invention relates to a process for analyzing, detecting or modulating nucleic acids by using said PNA oligomer.

[Description of Drawings]

Fig. 1 shows the structural difference of backbones of DNA and PNA;
Fig. 2 shows results and fluorescent images of PNA microarrays on which PCR products of HBV mutant type and wild type were hybridized with PNA probes comprising only natural bases and PNA probes comprising 5-nitroindole incorporated thereto.
Fig. 3 shows results and fluorescent images of PNA microarrays on which PCR products of HBV mutant type and wild type were hybridized with PNA probes comprising only natural bases and PNA probes comprising 3-nitropyrrole incorporated thereto.

Other and further objects, features and advantages of the invention will appear more fully from the following description.

[Best Mode]

Throughout the specification and claims of the invention, the term "a universal base"is defined as an artificial base which can be hybridized with at least two, more preferably four, bases, differently from a natural base such as adenine, thymine, guanine, cytosine and uracil.
In general, a PNA oligomer with high purine content has low water-solubility and decreased specificity in binding and increased non-specific binding with a target nucleic acid, thus to show low discriminability. Therefore, purine content in a base sequence is an important factor to be considered in the synthesisof a PNA oligomer. For example, it is difficult to manufacture a PNA oligomer containing at least 60% purine bases or at least four contiguous purine bases in its base sequence. It is also difficult to use such a PNA oligomer as a target-specific probe. Nevertheless, if a base sequence of a gene to be detected has pyrimidine-rich structure, an oligomer with high purine content has to be used as the probe. The present inventors noticed that substitution of some of the purines with universal bases could provide a desirable role to lower the purine content, as well as to remove the contiguity of purine bases.
Thus, the present invention is to increase water-solubility and to widen the application field of PNA oligomers, by substituting one or more purine bases with universal bases or inserting universal bases thereto to prevent decrease in water-solubility and non-specific hybridization, in case that there is a need for a PNA oligomer that contains at least 60% purine bases or at least four contiguous purine bases.
The PNA oligomer according to the present invention is represented by General Formula (1):

[General Formula 1]

$$A_p \quad B_q - C_r$$

, wherein $A_p$ and $C_r$ each represent a base sequence complementary to a base sequence other than a specific base sequence, e.g. in polymorphic or mutated region, of a target nucleic acid;

Bqrepresents a base sequence complementary to a specific base sequence, e.g. in polymorphic or mutated region, of a target nucleic acid;

wherein the target nucleic acid comprises the specific base sequence that contains at least 60% pyrimidine bases or at least four contiguous pyrimidine bases

p, q and r are the number of bases, p and r each represent an integer from 0 to 15, and q is an integer from 1 to 30, provided that p+q+r is an integer from 10 to 30; and

$A_p$-$B_q$-$C_r$ is a base sequence to which one or more universal bases capable of forming a base pair with natural DNA or RNA bases have been incorporated.

The PNA oligomer containing universal bases according to the invention makes it possible that various types of oligo-PNAs need not be synthesized for polymorphic regions. It also allows the use of a base sequence having an unidentified base without further analysis by substituting the base with a universal base. The present invention can also increase specificity by substituting a specific region of a probe with universal bases.

The PNA oligomers according to the invention have a wide variety of uses. For examples, they can be used in the field of DNA- or RNA-based therapy and diagnosis, and as probes of molecular biologyto improve their functions. For example, the PNA oligomer according to the invention can be used for detecting mutation or single nucleotide polymorphism in a target nucleotide sequence of a sample nucleic acid as well as indentifying species of a sample nucleic acid.

1) Preparation of PNA oligomers comprising universal bases In order to prepare a PNA oligomer comprising a universal base(s), a monomer must be used as in the synthesis of DNA oligomer, which can be incorporated to a PNA oligomer by a chemical process, and can form a base pair with a nucleotide moiety in its complementary strand, or enables appropriate base stacking within a nucleic acid strand. Thus, a PNA monomer comprising a universal base represented by Formula (4) was synthesized and used, as described in Hemavathi et al., 1999, Organic Letters Vol. 1, No. 10, 1639-1641.

[Formula 4]

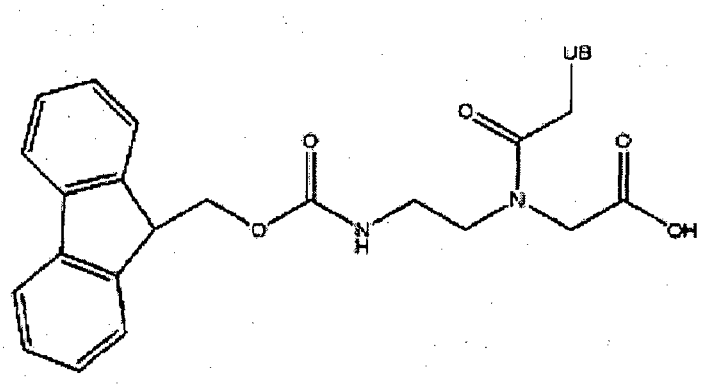

, wherein universal base (UB) is an artificial base to maximize base stacking while maintaining the structure of DNA double helix, for example, purine of formula (a), 3-nitropyrrole of formula (b), 5-nitroindole of formula (c), a benzimidazole compound of formula (d), benzene or fluorobenzene of formula (e), such as 4-fluorobenzene and pentafluorobenzene, xanthine of formula (f), a pyridopyrimidine compound of formula (g), a compound of formula (h) containing N or CH at X or Y position of the sugar chain, such as hypoxanthine, cytosinecompounds of formulas (i)~(k), an adenine compound of formula (1), 3-aminocarbonylpyrrole of formula (m), nitrodiazole of formula (n), a compound of formula (o) containing S or Se in the ring, a diazole compound of formula (p), a triazole compound of formula (q) or β-heptafluoronaphthalene of formula (r) (see US Patent 5,438,131 and Kathryn A. F et al., 2002, Chemical Communincation 2206-2207).

(a)

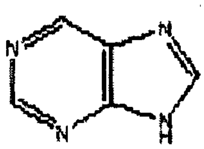

(b)

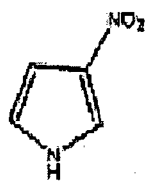

(c)

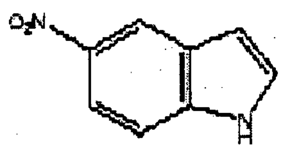

(d)

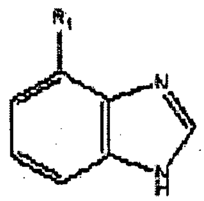

(e)

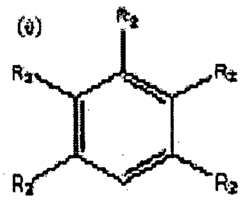

(f)

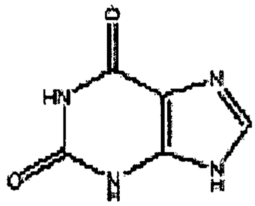

(g)

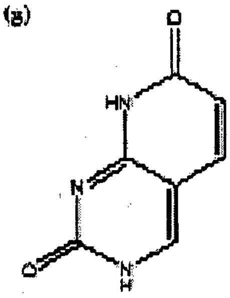

(h)

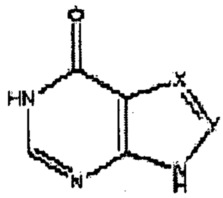

(i)

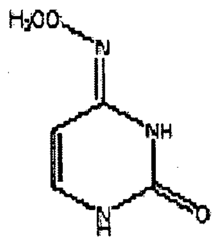

(j)

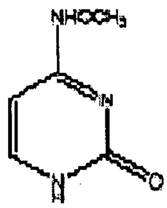

(k)

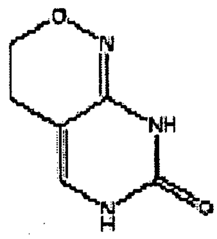

(l)

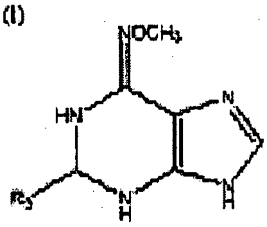

(m)

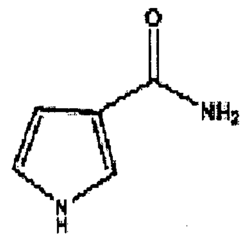

(n)

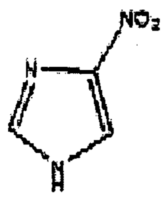

(o)

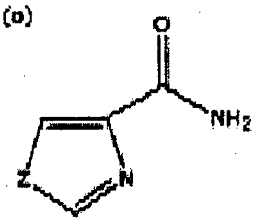

(p)

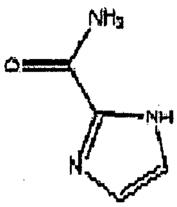

(q)

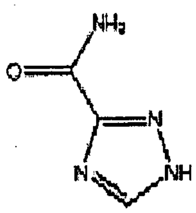

(r)

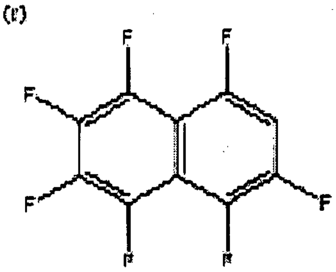

, wherein
$R_1$ is H, $NO_2$ or $NH_2$
$R_2$ is H or F;
$R_3$ is H or $NH_2$
X and Y independently from each other represent CH or N; and
Z is S or Se.

UB is preferably 3-nitropyrrole of formula (b) or 5-nitroindole of formula (c). Since these bases can bind to four types of bases (A, C, G and T) in a target nucleic acid with equivalent binding strength, they can reduce variation depending

upon the combination of mismatch pairs, to have been widely used for DNA oligomers.
In an embodiment of the present invention, the PNA oligomer consists of any one of base sequences represented by SEQ ID Nos. 2 to 7. The PNA oligomer of SEQ ID No. 1 is a 13mer consisting of natural bases to detect mutation. The PNA oligomer of SEQ ID No. 2 is a 13mer of $H_2N-A_6-B-C_6-COOH$, wherein the second base A of Region Cis substituted with 5-nitroindole. The PNA oligomer of SEQ ID No. 3 is a 14mer of $H_2N-A_6-B-C_7-COOH$, wherein the second base A of Region C is substituted with 5-nitroindole. The PNA oligomer of SEQ ID No. 4 is a 14mer of $H_2N-A_6-B-C_7-COOH$ like that of SEQ ID No. 3, wherein the fourth base A of Region Cis substituted with 5-nitroindole. The PNA oligomer of SEQ ID No. 5 is a 13mer of $H_2N-A_6-B-C_6-COOH$, wherein the second base A of Region Cis substituted with 3-nitropyrrole. The PNA oligomer of SEQ ID No. 6 is a 14mer of $H_2N-A_6-B-{}_C7-COOH$, wherein the second base A of Region Cis substituted with 3-nitropyrrole. The PNA oligomer of SEQ ID No. 7 is a 14mer of $H_2N-A_6-B-C_7-COOH$ like that of SEQ ID No. 6, wherein the fourth base A of Region Cis substituted with 3-nitropyrrole.

[Table 1]

| SEQ ID No. | Designation | Base sequence (NC) | Length (mer) |
|---|---|---|---|
| 1 | 180m-13 | GAGCCATGAGAAA | 13 |
| 2 | 180m+2x-13 | GAGCCATG$N_i$GAAA | 13 |
| 3 | 180m+2x-14 | GAGCCATG$N_i$GAAAC | 14 |
| 4 | 180m+4x-14 | GAGCCATGAG$N_i$AAC | 14 |
| 5 | 180m+2y-13 | GAGCCATG$N_p$GAAA | 13 |
| 6 | 180m+2y-14 | GAGCCATG$N_p$GAAAC | 14 |
| 7 | 180m+4y-14 | GAGCCATGAG$N_p$AAC | 14 |
| $N_i$: 5-nitroindole-PNA<br>$N_p$: 3-nitropyrrole-PNA | | | |

2) Application of PNA oligomers containing universal bases As described above, the PNA oligomers containing universal bases such as 5-nitroindole or 3-nitropyrrol have been studied for their hybridization properties heretofore. PNA oligomers containing universal bases have lower binding strength upon hybridizing with complementary DNA/RNA than natural bases, but have the property of binding with any of four types of bases. This property suggests that universal bases in PNA oligomers play the same role as ones in DNA oligomers, and universal bases are useful in PNA oligomers like in DNA oligomers and improve functions of a desired PNA oligomer upon incorporation to the PNA oligomer as to the DNA oligomer.

Thus, the PNA oligomers containing universal bases according to the invention are useful as oligomers for base sequence analysis based on hybridization, various probes in oligomer microarray, diagnosis of mutation, analysis of gene expression, detection of pathogenic microorganisms, probes for FISH, probes of Southern or Northern blot, oligomers of PCR clamping, probes of real-time PCR, antisense PNA oligomers, oligomers in diagnosis kit and tags for SAGE. Therefore, DNA oligomers can be substituted with more efficient PNA oligomers in the above technical fields. In addition, they can be applied as blocker probes for probe assay, or probes in mass spectroscopy.

The probes prepared from the PNA oligomers according to the invention may be labeledby a detectable marker, for exampleby fluorescence, and are preferably reacted in a solution. The PNA oligomers according to the invention can be immobilized onto glass, silica, semiconductor, magnetic particles, plastic, gold or silver tube or thin layer, a porous filter, or beads, for use in the form of chip.

The PNAs containing universal bases according to the invention can be immobilized onto the surface of a functionalized solid support to be manufactured into a device for detecting or analyzing base sequence of nucleic acid. These devices include, but are not limited to, a PNA microarray or a PNA chip with a number of PNA probes arrayed in 2-dimension, microbeads of several micrometer size having PNA probes immobilized onto the surface thereof, a field-effect transistor comprising PNA probes immobilized onto silicon semiconductor or silicon nanowire, an impedance detector, a microcantilever, a surface acoustic wave (SAW) sensor, and the like.

Examples

Hereinafter, the present invention will be explained in more detail with reference to specific examples, which are provided only for better understanding of the invention, but should not be construed to limit the scope of the invention in any manner. Preparation: Synthesis of PNA monomers containing universal bases

**Preparation 1: Synthesis of 2-N-(2-((9H-fluoren-9-yl)methoxy)carbonylamino)ethyl)-2-(3-nitro-1H-pyrrol-1-yl)acetamido)acetic acid**

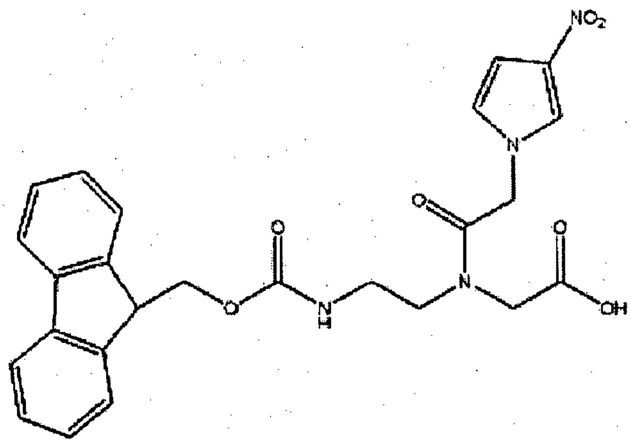

The title compound was synthesized according to the procedure as reported by H. Challa [H. Challa et al., Organic Letters 1999, Vol 1, p1639].
$^1$H NMR(DMSO-d6): 7.82 (d, 2H), 7.75 (s, 1H), 7.51(d, 2H), 7.34 (t, 2H), 7.25 (t, 2H), 6.70 (m, 1H), 6.56(m, 1H), 5.01/4.81 (rotomer, s, 2H), 4.30-4.13 (m, 3H), 4.14/3.92 (rotomer, s, 2H), 3.40-3.04 (m, 4H).

**Preparation 2: Synthesis of 2-(N-(2-(((9H-fluoren-9-yl)methoxy)carbonylamino)ethyl)-2-(5-nitro-1H-indol-1-yl)acetamido)acetic acid**

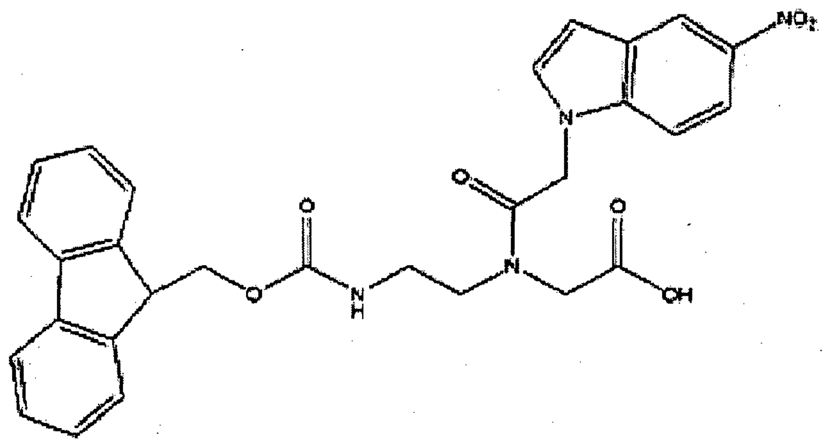

The title compound was synthesized according to the procedure as reported by H. Challa [H. Challa et al., Organic Letters 1999, Vol 1, p1639].
$^1$H NMR(DMSO-d6): 8.57 (s, 1H), 7.97 (d, 1H), 7.82 (d, 2H), 7.70-7.22(m, 7H), 6.74 (d, 1H), 5.41/5.14 (rotomer, s, 2H), 4.35-4.00 (m, 5H), 3.66-3.24 (m, 4H).

**Examples 1 to 6 and Comparative Example 1: Preparation of PNA oligomers containing universal bases and natural bases**

PNA oligomers containing universal bases and natural bases were prepared from PNA monomers containing universal bases and natural bases according to conventionalsolid-phase synthetic method. Fmoc-Lys(Fmoc)-OH and O linker {2-[2-(9H-fluoren-9-ylmethoxycarbonylamino)ethoxy]ethoxy}acetic acid were used as linkers to manufacture an

array. That is, PNA oligomers were synthesized from PNA monomers containing benzothiazolsulfonyl group for protecting amine in the backbone of PNA and benzohydrylcarbonyl group for protecting exocyclic amine in the nucleobase, according to the procedure as described in Korean Patent Registration No. 555204.

First, 10 equivalents of a PNA monomer or a linker was mixed with 9.5 equivalents of 2-(1H-benzotriazol-1-yl)-1,1,3,3-tetramethyluronium hexafluorophosphate (HBTU), 19 equivalents of N,N-diisopropylethylamine (DIEA) and dimethylformamide (DMF), and activated for 10 minutes. The resultant solution was added to resin to which a PNA containing an amine group at the N-terminal had been attached, and the mixture was shaken at room temperature for 60 minutes. The resin was washed with DMF three times, and subjected to capping with a solution of 5% acetic anhydride and 6% lutidine inDMF for 5 minutes. The mixture was washed with DMF three times, and reacted in a solution of 2 M piperidine in DMF for 10 minutes to remove the Fmoc protective group. Then, the mixture was washed with DMF three times. Upon completion of the coupling of a PNA monomer as described above, the rest of PNA oligomer was synthesized according to the procedure as described in Korean Patent Registration No. 555204. Upon completion of the synthesis of PNA oligomer, the end benzothiazole-2-sulfonyl group was removed, and the mixture was treated with trifluoroacetic acid containing 25% m-cresol for 1.5 hours to separate the PNA oligomer from the resin. The PNA oligomer obtained was purified by HPLC and lyophilized.

According to the procedure described above, PNA oligomers of Examples 1 to 6 and Comparative Example 1 were synthesized. The base sequences were to detect point mutations of lamivudine-resistant mutant types of hepatitis B virus (HBV).

[Table 2]

| Ex./ Comp.Ex. | SEQ ID No. | Designation | Base sequence (N->C) | MS Calculated (M) | MS Found (M+1) |
|---|---|---|---|---|---|
| Comp.Ex.1 | 1 | 180m-13 | k(k)k-OO-GAGCCATGAGAAA | 4277.3 | 4277.8 |
| Ex.1 | 2 | 180m+2x-13 | k(k)k-OO-GAGCCATG$N_i$GAAA | 4306.3 | 4307.5 |
| Ex.2 | 3 | 180m+2x-14 | k(k)k-OO-GAGCCATG$N_i$GAAAC | 4557.6 | 4558.3 |
| Ex.3 | 4 | 180m+4x-14 | k(k)k-OO-GAGCCATGAG$N_i$AAC | 4557.6 | 4558.5 |
| Ex.4 | 5 | 180m+2y-13 | k(k)k-OO-GAGCCATG$N_p$GAAA | 4002.0 | 4002.9 |
| Ex.5 | 6 | 180m+2y-14 | k(k)k-OO-GAGCCATG$N_p$GAAAC | 4253.2 | 4254.8 |
| Ex.6 | 7 | 180m+4y-14 | k(k)k-OO-GAGCCATGAG$N_p$AAC | 4253.2 | 4254.9 |
| k: L-lysine, O: 8-amino-3,6-dioxaoctanoic acid, $N_i$: 5-nitroindole-PNA, $N_p$: 3-nitropyrrole-PNA | | | | | |

**Example 7: Synthesis of primers for preparing target nucleic acid**

For the preparation of target DNA according to the invention, primers for PCR were synthesized. The primer sequence was chosen by analyzing ones that can react with lamivudine resistant gene of HBV, as shown in Table 3. The PCR primers had biotin attached at their 5'-terminal.

[Table 3]

| Primer | SEQ ID No. | Base Sequence (NC) | Remark |
|---|---|---|---|
| Hbv-F | 8 (sense) | CCA TCA TCT TGG GCT TTC GC | Biotin attached |
| Hbv-R | 9(antisense) | CAA AAG AAA ATT GGT AAC AGC GGT A | Biotin attached |

**Example 8: Preparation of target nucleic acid by PCR**

Thegene associated with resistance to lamivudine, a therapeutic agent for chronic HBV infection, was cloned and the cloned DNA was employed. Under the condition as shown in Table 4, the reaction compositions were prepared and PCR were carried out for HBV mutant type and wild type, respectively.

[Table 4]

| Reaction composition (μℓ) | | Reaction temperature | Cycles |
|---|---|---|---|
| Sterilized distilled water | 37.6 | 95°C, 5.0 min | 1 |
| 10-fold buffer | 5 | | |
| 2 mM dNTP | 3 | 0.5 min | 30 |
| 10 pmol/μℓ Sense primer | 1 | 95°C, 1.0 min | 30 |
| 10 pmol/μℓ Antisense primer | 1 | 55°C, 0.5 min | 30 |
| 1 μ Taq | 0.4 | 72°C, 1.0 min | 30 |
| Template | 2 | 72°C, 7.0 min | 1 |
| Total | 50 | | |

Upon completion of thereaction, to the PCR product of 5 μℓ was added a gel loading buffer of 1 μl, and the mixture was subjected to electrophoresis on 1.5% agarose gel, and stained with 1 μg/ml of ethidium bromide (EtBr), and the product was observed under a UV-transilluminator.

**Example 9: Manufacture of PNA microarray**

The purified oligomer was diluted at a concentration of 50 mM, and spotted on a slide glass functionalized with epoxy group (Panagene, Korea) in pin mode, and the slide was allowed to stand for 4 hours at room temperature while maintaining 75% humidity. Then, it was immersed into DMF, and washed by ultrasonication for 15 minutes. It was immersed into DMF containing 0.1 M succinic anhydride and reacted at 40°C for 2 hours. And the reaction mixture was washed by ultrasonication with DMF and deionized water, sequentially, for 15 minutes per time. Then, the slide was treated with 100 mM Tris-HCl buffer containing 0.1 M ethanolamine for 2 hours to inactivate the residual epoxy groups on the surface of the slide glass. The slide glass was washed by ultrasonication with deionized water, twice for 15 minutes per time, treated with boiling water for 5 minutes, washed with deionizedwater for 5 minutes, and dried. Then, a PNA microarray was ready for hybridization after attaching a silicon rubber sealing mat having holes, which can contain 100 μℓ of hybridization solution, to the slide glass.

**Example 10: Hybridization with target nucleic acid**

To a hybridization solution of 100 μℓ was added 5 μℓ of the PCR product of HBV mutant type having biotinattached thereto, and streptavidine-Cy5 was added thereto to cause fluorescent reaction. The hole of silicone rubber sealing mat on the slide glass was filled with the hybridization mixture of 100 μl, and the reaction was performed at 40°C for 2 hours. Upon completion of the reaction, the slide was washed twice with washing buffer at room temperature for 5 minutes, and dried. The PNA microarray was scanned by a fluorescence scanner. The same experiment was carried out for the PCR product of HBV wild type.

The results are shown in Figs. 2 and 3. As can be seen from Figs. 2 and 3, the discriminability between perfect match signal and single mismatch signal was increased by incorporating universal bases or substituting some of contiguous purine bases for universal bases or incorporating universal basesin the PNA oligomer containing at least 60% purine bases (G, A) or contiguous purine bases. Specifically, the PNA probe of SEQ ID No. 1, the PNA oligomer wherein six contiguous purine bases (GAGAAA) are present, shows intensive single mismatch signal for the PCRproduct of HBV wild type as well as intensive perfect match signal for the PCR product of HBV mutant type, so that it cannot discriminate HBV mutant type against wild type. On the contrary, the PNA probes (SEQ ID Nos. 2 to 4) wherein the second or fourth A among those six contiguous purine bases was replaced by 5-nitroindole, showed decreased single mismatch signal as compared to the PNA oligomer of SEQ ID No. 1, thereby increasing the perfect match/mismatch (P/M) ratio (see Fig. 2). The PNA probes (SEQ ID Nos. 5 to 7), wherein the second or fourth A among those six contiguous purine bases was replaced by 3-nitropyrrole, also showed increased P/M ratio indicating discriminability between perfect match signal for the PCR product of HBV mutant type and single mismatch signal for the PCR product of HBV wild type, as compared to the PNA probe of SEQ ID No. 1, so that they could detect HBV mutant type specifically (Fig. 3).

[Industrial Applicability]

In preparing PNA oligomers with desired base sequences, the present invention controls the purine content,

thereby preventing decrease in water-solubility and decrease in specificity upon hybridization by eliminating non-specific hybridization of purine-rich PNA oligomers with nucleic acids. Thus, the present invention can widen the applicability of PNA oligomer, e.g. for analyzing or detecting a specific gene or base sequence based on hybridization, or for modulating, i.e. promoting or inhibiting, functions thereof.

[Sequence List Text]

SEQ ID No. 1 is the base sequence of the PNA oligomer consisting of natural bases;
SEQ ID No.2 is the base sequence of the PNA oligomer of $H_2N-A_6-B-C_6-COOH$, wherein the second base A of Region Cis substituted with 5-nitroindole;
SEQ ID No. 3 is the base sequence of the PNA oligomer of $H_2N-A_6-B-C_7-COOH$, wherein the second base A of Region Cis substituted with 5-nitroindole;
SEQ ID No. 4 is the base sequence of the PNA oligomer of $H_2N-A_6-B-C_7-COOH$, wherein the fourth base A of Region Cis substituted with 5-nitroindole;
SEQ ID No. 5 is the base sequence of the PNA oligomer consisting of $H_2N-A_6-B-C_6-COOH$, wherein the second base A of Region Cis substituted with 3-nitropyrrole;
SEQ ID No. 6 is the base sequence of the PNA oligomer consisting of $H_2N-A_6-B-C_7-COOH$, wherein the second base A of Region C is substituted with 3-nitropyrrole;
SEQ ID No. 7 is the base sequence of the PNA oligomer consisting of $H_2N-A_6-B-C_7-COOH$, wherein the fourth base A of Region Cis substituted with 3-nitropyrrole;
SEQ ID No. 8 is the base sequence of the forward primer to amplify the region of lamivudine-resistant gene of HBV and SEQ ID No. 9 is the base sequence of the reverse primer to amplify the region of lamivudine-resistant gene of HBV.

## Claims

1. A peptide nucleic acid (PNA) oligomer with increased solubility in water and specificity upon hybridization with nucleic acid, which comprises at least one universal base, capable of forming a base pair with natural DNA or RNA bases, incorporated in its base sequence, the base sequence having at least 60% purine bases or at least four contiguous purine bases and which consists of the base sequence represented by General Formula (1):

[Formula 1]

$$A_p\ B_q - C_r,$$

wherein $A_p$ and $C_r$ each represent a base sequence complementary to a base sequence other than a specific base sequence of a target nucleic acid;
Bq represents a base sequence complementary to a specific base sequence of a target nucleic acid;
wherein the target nucleic acid comprises the specific base sequence that contains at least 60% pyrimidine bases or at least four contiguous pyrimidine bases
p, q and r are the number of bases, p and r each represent an integer from 0 to 15, and q is an integer from 1 to 30, provided that p+q+r is an integer from 10 to 30; and
$A_p$-$B_q$-$C_r$ is a base sequence to which one or more universal bases, capable of forming a base pair with natural DNA or RNA bases, have been incorporated.

2. The PNA oligomer according to claim 1, wherein the specific base sequence of the target nucleic acid is of polymorphic or mutated region.

3. The PNA oligomer according to claim 1, wherein the universal base is one or more selected from the group consisting of Formulas (a) to (r):

(a)

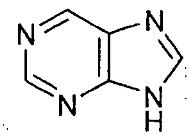

(b)

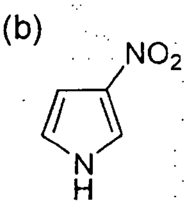

(c)

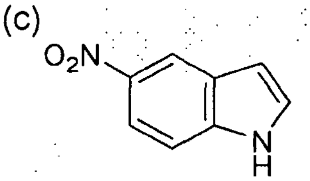

(d)

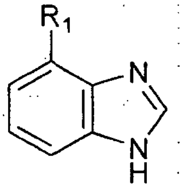

(e)

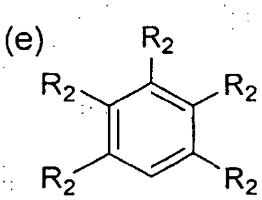

(f)

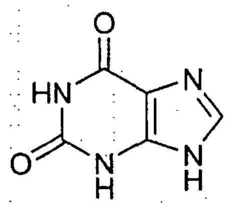

(g)

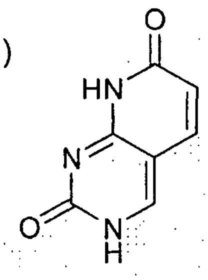

(h)

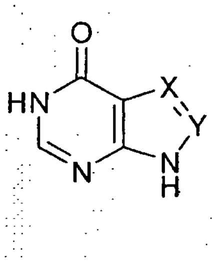

(i)

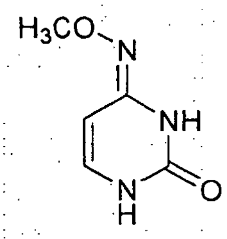

(j)

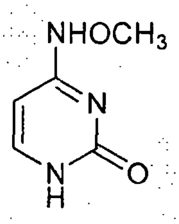

(k)

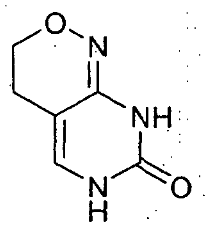

(l)

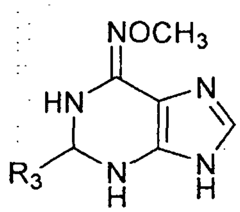

(m)

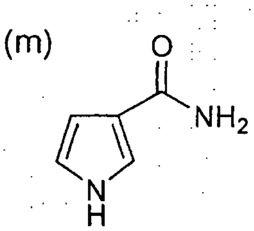

(n)

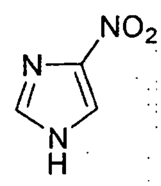

(o)

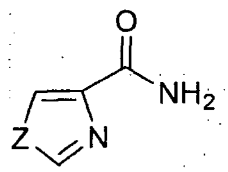

(p) 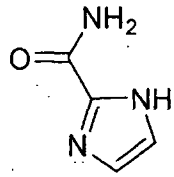 (q) 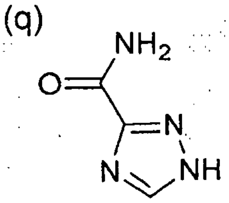 (r) 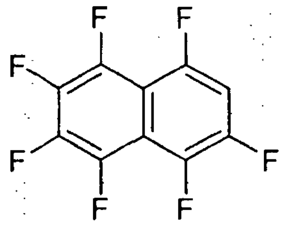 ,

wherein

*182$R_1$ is H, $NO_2$ or $NH_2$;
$R_2$ is H or F;
$R_3$ is H or $NH_2$;
X and Y independently from each other represent CH or N; and Z is S or Se.

**4.** The PNA oligomer according to claim 3, wherein said universal base is the group of formula (b) or (c).

**5.** The PNA oligomer according to claim 4, which consists of any one of base sequences selected from SEQ ID NOs: 2 to 7.

**6.** A probe comprising the PNA oligomer according to any one of claims 1 to 5.

**7.** The probe according to claim 6, for use in PNA array, FISH, Southern blot, Northern blot, real time-PCR, SAGE, PCR clamping, a kit for diagnosis, probe assay or mass spectroscopy.

**8.** The probe according to claim 6, which is labeled with a detectable marker.

**9.** The probe according to claim 6, wherein the PNA oligomer is used in a solution.

**10.** A method of preparing a PNA oligomer according to claim 1 with increased solubility in water and specificity upon hybridization with nucleic acid, which comprises the steps of:

1) examining whether the PNA oligomer to be designed comprises at least 60% purine bases or at least four contiguous purine bases in its base sequence;
2) synthesizing PNA monomers comprising natural bases and universal bases, respectively; and
3) incorporating at least one universal base, capable of forming a base pair with natural DNA or RNA bases, to the base sequence with said PNA monomer, to prepare the PNA oligomer.

**11.** A kit for analyzing, detecting or modulating nucleic acid, which comprises the PNA oligomer according to any one of claims 1 to 5.

**12.** The kit for analyzing, detecting or modulating nucleic acid according to claim 11, wherein the PNA oligomer is immobilized onto glass, silica, magnetic particles, semiconductor, plastic, gold or silver tube or thin film, a porous filter or beads.

**13.** A device for analyzing or detecting nucleic acid, which comprises the PNA oligomer according to any one of claims 1 to 5.

**14.** The device for analyzing or detecting nucleic acid according to claim 13, wherein the PNA oligomer is immobilized onto glass, silica, magnetic particles, semiconductor, plastic, gold or silver tube or thin film, a porous filter or beads.

**15.** A method of analyzing or detecting nucleic acid by using the PNA oligomer according to any one of claims 1 to 5.

## Patentansprüche

1. Peptidnucleinsäure(PNA)-Oligomer mit erhöhter Löslichkeit in Wasser und Spezifität bei Hybridisierung mit Nucleinsäure, das wenigstens eine universelle Base umfasst, die zur Bildung eines Basenpaares mit natürlichen DNA- oder RNA-Basen, die in seine Basensequenz eingebaut sind, fähig ist, wobei die Basensequenz wenigstens 60% Purinbasen oder wenigstens vier fortlaufende Purinbasen hat, und das aus der Basensequenz besteht, die durch die allgemeine Formel (1) dargestellt wird:

[Formel 1]

$$A_p \ B_q - C_r,$$

worin $A_p$ und $C_r$ jeweils eine Basensequenz darstellen, die zu einer anderen Basensequenz als einer spezifischen Basensequenz einer Target-Nucleinsäure komplementär ist;
$B_q$ eine zu einer spezifischen Basensequenz einer Target-Nucleinsäure komplementäre Basensequenz darstellt; wobei die Target-Nucleinsäure die spezifische Basensequenz umfasst, die wenigstens 60% Pyrimidinbasen und wenigstens vier fortlaufende Pyrimidinbasen enthält;
p, q und r die Zahl an Basen sind, p und r jeweils eine ganze Zahl von 0 bis 15 darstellen und q eine ganze Zahl von 1 bis 30 ist, mit der Maßgabe, dass p+q+r eine ganze Zahl von 10 bis 30 ist, und
$A_p$-$B_q$-$C_r$ eine Basensequenz ist, in die eine oder mehrere universelle Base(n), die zur Bildung eines Basenpaares mit natürlichen DNA- oder RNA-Basen fähig ist/sind, eingebaut ist/sind.

2. PNA-Oligomer nach Anspruch 1, wobei die spezifische Basensequenz der Target-Nucleinsäure aus polymorpher oder mutierter Region ist.

3. PNA-Oligomer nach Anspruch 1, wobei die universelle Base eine oder mehrere, ausgewählt aus der Gruppe, bestehend aus den Formeln (a) bis (r), ist:

(a)
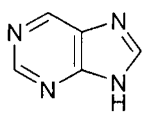

(b)
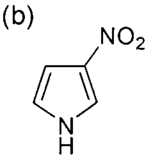

(c)
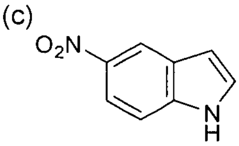

(d)
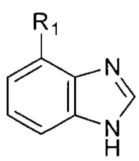

(e)
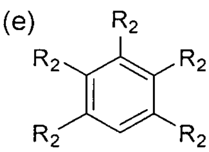

(f)
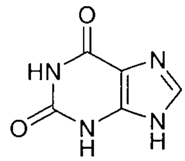

(g)
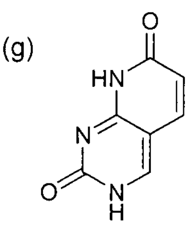

(h)
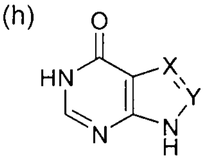

(i)
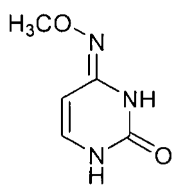

(j) 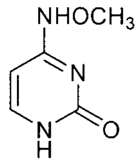

(k) 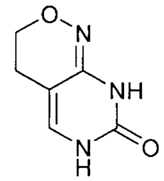

(l) 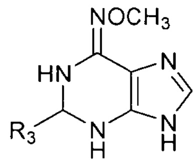

(m) 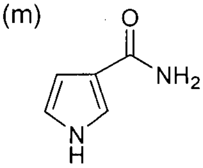

(n) 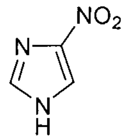

(o) 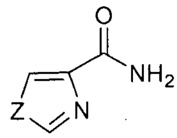

(p) 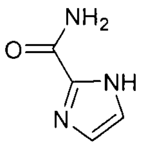

(q) 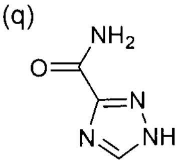

(r) 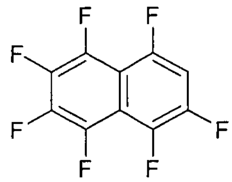

,

worin

*182$R_1$ für H, $NO_2$ oder $NH_2$ steht;
$R_2$ für H oder F steht;
$R_3$ für H oder $NH_2$ steht;
X und Y unabhängig voneinander CH oder N darstellen und Z für S oder Se steht.

**4.** PNA-Oligomer nach Anspruch 3, wobei die universelle Base die Gruppe der Formel (b) oder (c) ist.

**5.** PNA-Oligomer nach Anspruch 4, das aus einer der Basensequenzen, ausgewählt aus SEQ ID NOs: 2 bis 7, besteht.

**6.** Sonde, die das PNA-Oligomer nach einem der Ansprüche 1 bis 5 umfasst.

**7.** Sonde nach Anspruch 6 zur Verwendung in einem PNA-Array, einem FISH-Test, Southern Blot, Northern Blot, Echtzeit-PCR, einer SAGE, PCR-Clamping, einem Kit zur Diagnose, einem Sondenessay oder in der Massenspektroskopie.

**8.** Sonde nach Anspruch 6, welche mit einem detektierbaren Marker markiert ist.

**9.** Sonde nach Anspruch 6, wobei das PNA-Oligomer in einer Lösung verwendet wird.

**10.** Verfahren zur Herstellung eines PNA-Oligomers nach Anspruch 1 mit erhöhter Löslichkeit in Wasser und Spezifität bei Hybridisierung mit Nucleinsäure, das die Schritte:

1) Untersuchen, ob das zu entwickelnde PNA-Oligomer wenigstens 60% Purinbasen oder wenigstens vier fortlaufende Purinbasen in seiner Basensequenz enthält;
2) Synthetisieren, von PNA-Monomeren, die natürliche Basen bzw. universelle Basen umfassen, und
3) Einbauen wenigstens einer universellen Base, die zur Bildung eines Basenpaares mit natürlichen DNA- oder RNA-Basen fähig ist, in die Basensequenz mit dem PNA-Monomer, um das PNA-Oligomer herzustellen, umfasst.

**11.** Kit zum Analysieren, Detektieren oder Modulieren einer Nucleinsäure, der das PNA-Oligomer nach einem der Ansprüche 1 bis 5 umfasst.

**12.** Kit zum Analysieren, Detektieren oder Modulieren einer Nucleinsäure nach Anspruch 11, wobei das PNA-Oligomer

an Glas, Siliciumdioxid, magnetischen Teilchen, Halbleiter, Kunststoff, Gold- oder Silberröhre oder Dünnfilm, einem porösen Filter oder Beads immobilisiert ist.

**13.** Vorrichtung zum Analysieren oder Detektieren von Nucleinsäure, die das PNA-Oligomer nach einem der Ansprüche 1 bis 5 umfasst.

**14.** Vorrichtung zum Analysieren oder Detektieren von Nucleinsäure nach Anspruch 13, wobei das PNA-Oligomer an Glas, Siliciumdioxid, magnetischen Teilchen, Halbleiter, Kunststoff, Gold- oder Silberröhre oder Dünnfilm, einem porösen Filter oder Beads immobilisiert ist.

**15.** Verfahren zum Analysieren oder Detektieren von Nucleinsäure durch Verwendung des PNA-Oligomers nach einem der Ansprüche 1 bis 5.

## Revendications

**1.** Oligomère d'acide nucléique peptidique (ANP) présentant une augmentation de solubilité dans l'eau et de spécificité après hybridation avec un acide nucléique, qui comprend au moins une base universelle, capable de former une paire de bases avec des bases naturelles d'ADN ou d'ARN, incorporée dans sa séquence de bases, la séquence de bases comportant au moins 60 % de bases purine ou au moins quatre bases purine contiguës et qui est constitué de la séquence de bases représentée par la formule générale (1) :

$$A_p\ B_q - C_r$$

dans laquelle $A_p$ et $C_r$ représentent chacun une séquence de bases complémentaire d'une séquence de bases autre qu'une séquence de bases spécifique d'un acide nucléique cible ;
$B_q$ représente une séquence de bases complémentaire d'une séquence de bases spécifique d'un acide nucléique cible ;
où l'acide nucléique cible comprend la séquence de bases spécifique qui contient au moins 60 % de bases pyrimidine ou au moins quatre bases pyrimidine contiguës
p, q et r représentent le nombre de bases, p et r représentent chacun un nombre entier de 0 à 15, et q est un nombre entier de 1 à 30, à condition que p+q+r soit un nombre entier de 10 à 30 ; et
$A_p$-$B_q$-$C_r$ représente une séquence de bases dans laquelle une ou plusieurs bases universelles, capables de former une paire de bases avec des bases naturelles d'ADN ou d'ARN, ont été incorporées.

**2.** Oligomère d'ANP selon la revendication 1, dans lequel la séquence de bases spécifique de l'acide nucléique cible est d'une région polymorphe ou mutée.

**3.** Oligomère d'ANP selon la revendication 1, dans lequel la base universelle est une ou plusieurs choisies dans le groupe constitué des formules (a) à (r) :

(a)
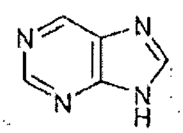

(b)
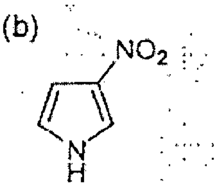

(c)
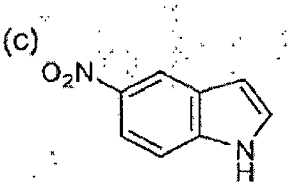

(d)
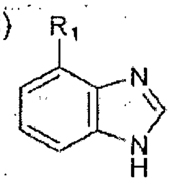
(e)
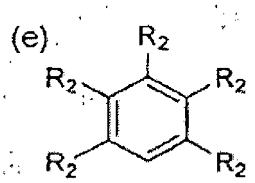
(f)
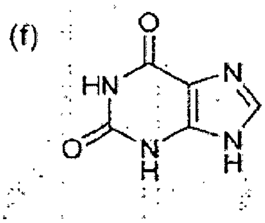

(g)
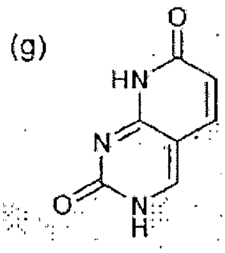
(h)
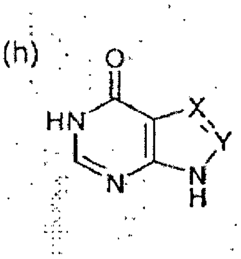
(i)
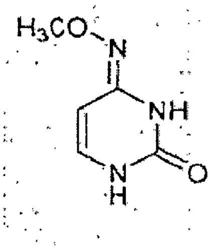

(j)
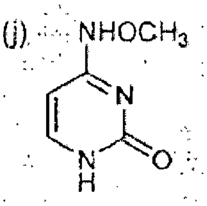
(k)
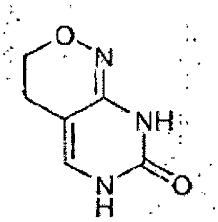
(l)
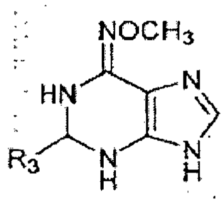

(m)
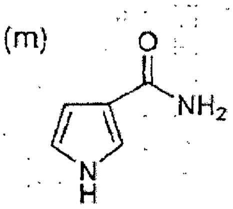
(n)
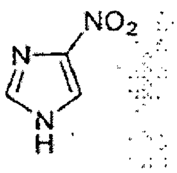
(o)
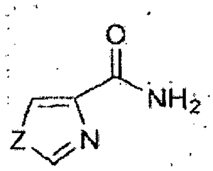

(p)
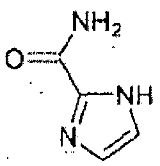
(q)
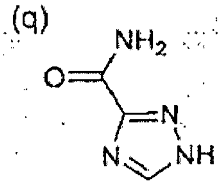
(r)
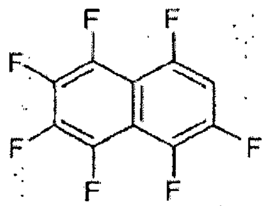

où

*182$R_1$ représente H, $NO_2$ ou $NH_2$ ;
$R_2$ représente H ou F ;
$R_3$ représente H ou $NH_2$ ;
X et Y chacun indépendamment l'un de l'autre représentent CH ou N ; et
Z représente S ou Se.

**4.** Oligomère d'ANP selon la revendication 3, dans lequel ladite base universelle est le groupe de formule (b) ou (c).

**5.** Oligomère d'ANP selon la revendication 4, qui est constitué de l'une quelconque des séquences de bases choisies

parmi SEQ ID NO : 2 à 7.

6. Sonde comprenant l'oligomère d'ANP selon l'une quelconque des revendications 1 à 5.

7. Sonde selon la revendication 6, pour une utilisation dans une puce d'ANP, une technique FISH, Southern blot, Northern blot, PCR en temps réel, SAGE, PCR clamping, un kit de diagnostic, un test à base de sondes ou une spectrométrie de masse.

8. Sonde selon la revendication 6, qui est marquée avec un marqueur détectable.

9. Sonde selon la revendication 6, où l'oligomère d'ANP est utilisé dans une solution.

10. Procédé de préparation d'un oligomère d'ANP selon la revendication 1 présentant une augmentation de solubilité dans l'eau et de spécificité après hybridation avec un acide nucléique, qui comprend les étapes suivantes :

    1) l'étude si l'oligomère d'ANP à concevoir comprend au moins 60 % de bases purine ou au moins quatre bases purine contiguës dans sa séquence de bases ;
    2) la synthèse de monomères d'ANP comprenant des bases naturelles et des bases universelles, respectivement ; et
    3) l'incorporation d'au moins une base universelle, capable de former une paire de bases avec des bases naturelles d'ADN ou d'ARN, dans la séquence de bases avec ledit monomère d'ANP, pour préparer l'oligomère d'ANP.

11. Kit pour l'analyse, la détection ou la modulation d'un acide nucléique, qui comprend l'oligomère d'ANP selon l'une quelconque des revendications 1 à 5.

12. Kit pour l'analyse, la détection ou la modulation d'un acide nucléique selon la revendication 11, dans lequel l'oligomère d'ANP est immobilisé sur du verre, de la silice, des particules magnétiques, un semi-conducteur, du plastique, de l'or ou un tube en argent ou un film mince, un filtre poreux ou des billes.

13. Dispositif pour l'analyse ou la détection d'un acide nucléique, qui comprend l'oligomère d'ANP selon l'une quelconque des revendications 1 à 5.

14. Dispositif pour l'analyse ou la détection d'un acide nucléique selon la revendication 13, dans lequel l'oligomère d'ANP est immobilisé sur du verre, de la silice, des particules magnétiques, un semi-conducteur, du plastique, de l'or ou un tube en argent ou un film mince, un filtre poreux ou des billes.

15. Procédé d'analyse ou de détection d'un acide nucléique en utilisant l'oligomère d'ANP selon l'une quelconque des revendications 1 à 5.

【Figure 1】

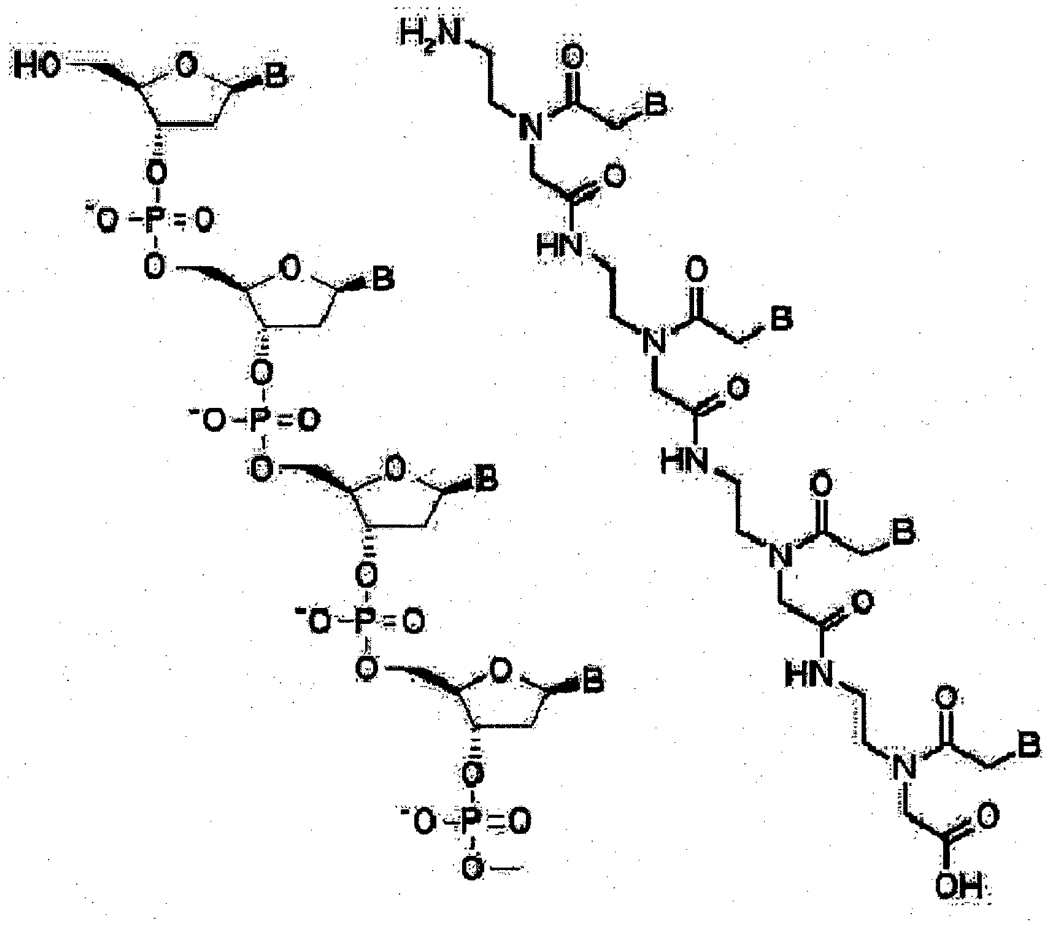

DNA

PNA

【Figure 2】

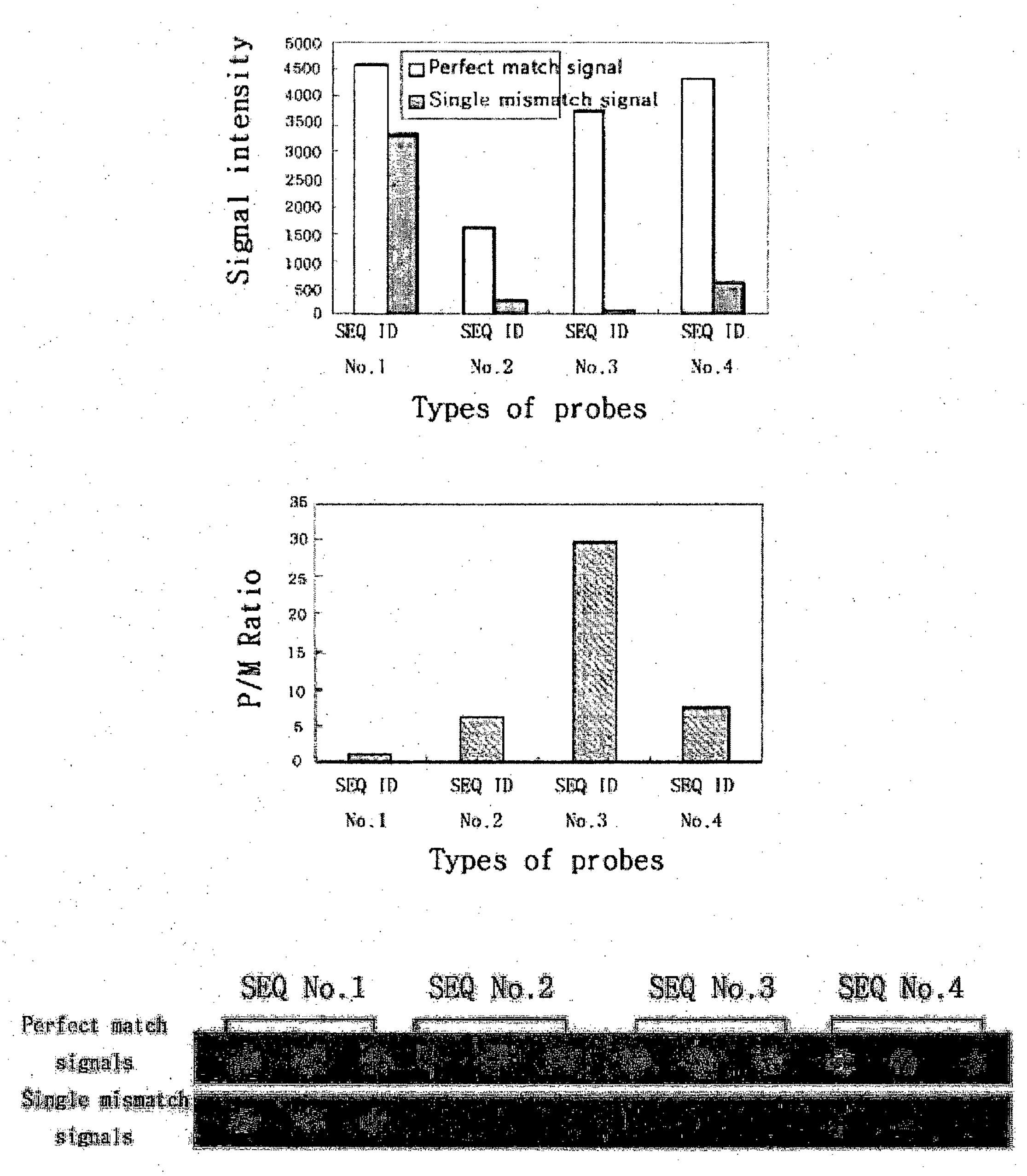
Signal intensity
5000
4500
4000
3500
3000
2500
2000
1500
1000
500
0
Perfect match signal
Single mismatch signal
SEQ ID No.1
SEQ ID No.2
SEQ ID No.3
SEQ ID No.4
Types of probes
P/M Ratio
35
30
25
20
15
10
5
0
SEQ ID No.1
SEQ ID No.2
SEQ ID No.3
SEQ ID No.4
Types of probes
SEQ No.1
SEQ No.2
SEQ No.3
SEQ No.4
Perfect match signals
Single mismatch signals

【Figure 3】

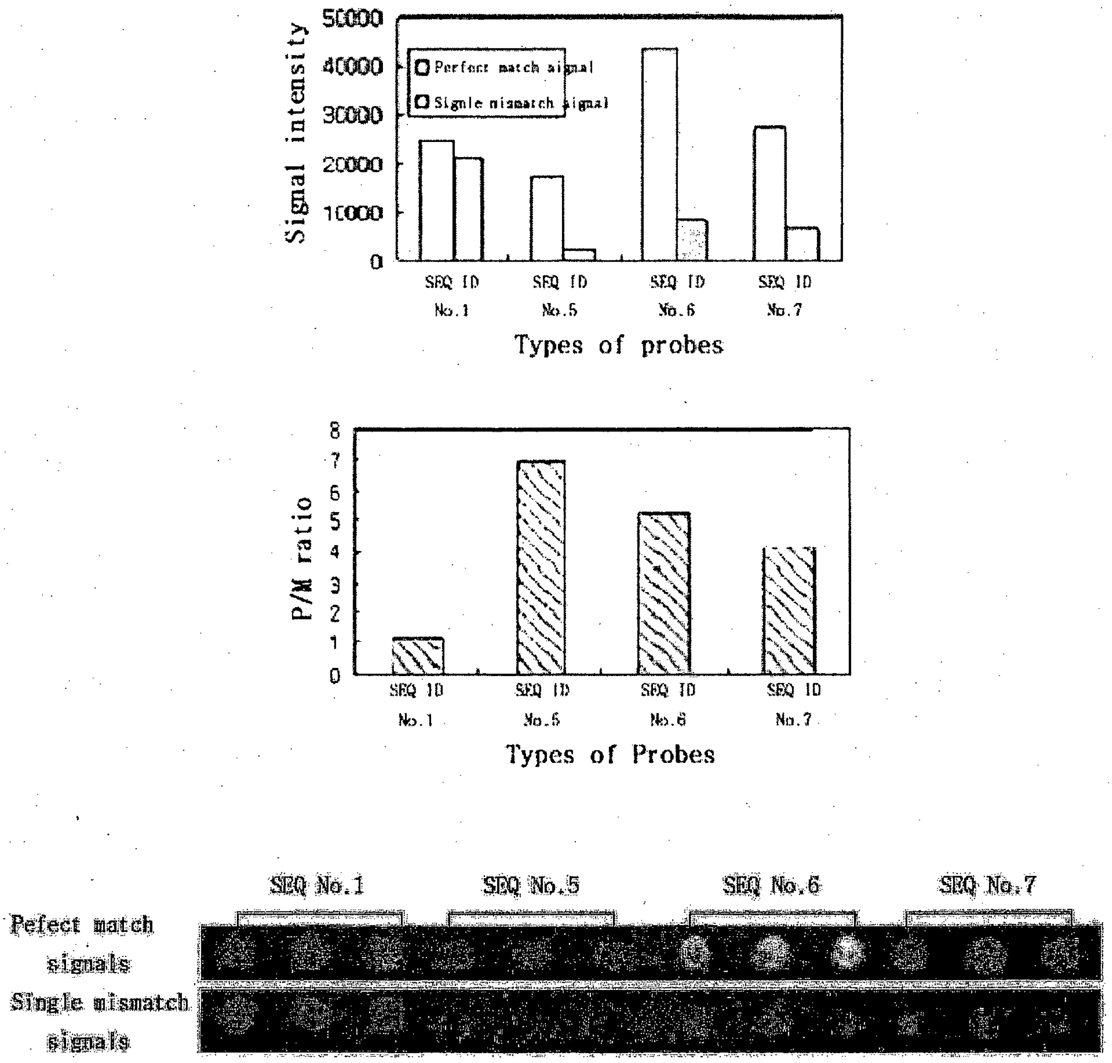
Signal intensity
50000
40000
30000
20000
10000
0
Perfect match signal
Single mismatch signal
SEQ ID No.1
SEQ ID No.5
SEQ ID No.6
SEQ ID No.7
Types of probes
P/M ratio
8
7
6
5
4
3
2
1
0
SEQ ID No.1
SEQ ID No.5
SEQ ID No.6
SEQ ID No.7
Types of Probes
SEQ No.1
SEQ No.5
SEQ No.6
SEQ No.7
Pefect match signals
Single mismatch signals

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7282329 B, S. Manalis and T. Burg
- US 20040072208 A1, P. Warthoe and S. Iben
- US 5438131 A
- KR 555204
- EP 07851574 A
- KR 1020060129517


### Non-patent literature cited in the description


- **NIELSEN PE ; EGHOLM M ; BERG RH ; BUCHARDT O.** Sequence-selective recognition of DNA by strand displacement with a thymine-substituted polyamide. *Science,* 1991, vol. 254, 1497-1500
- **BRANDT O ; HOHEISEL JD.** Peptide nucleic acids on microarrays and other biosensors. *Trends Biotechnology,* 2004, vol. 22, 617-622
- **ROCKENBAUER E ; PETERSEN KH ; VOGEL U ; BOLUND L ; KØLVRAA S ; NIELSEN KV ; NEXØ BA.** SNP genotyping using microsphere-linked PNA and flow cytometric detection. *Cytometry,* 2005, vol. 64, 80-86
- **F. USLU et al.** Labelfree fully electronic nucleic acid detection system based on a field-effect transistor device. *Biosensors and Bioelectronics,* 2004, vol. 19, 1723-1731
- **J. HAHM ; C. M. LIEBER.** Direct ultrasensitive electrical detection of DNA and DNA sequence variations using nanowire nanosensors. *Nano Letters,* 2004, vol. 4, 51-54
- **A. MACANOVIC et al.** Impedance-based detection of DNA sequences using a silicon transducer with PNA as the probe layer. *Nucleic Acids Research,* 2004, vol. 32, 20
- **JAN WEILER et al.** Hybridisation based DNA screening on peptide nucleic acid (PNA) oligomer arrays. *Nucleic Acids Research,* 1997, vol. 25 (14), 2792-2799
- **A. J. TACKETT et al.** Non-Watson-Crick interactions between PNA and DNA inhibit the ATPase activity of bacteriophase T4 Dda helicase. *Nucleic Acids Research,* 2002, vol. 30, 950-957
- **T. A. MILLICAN et al.** *Nucleic Acids Research,* 1984, vol. 12, 7435-7453
- **R. ERITJA et al.** *Nucleic Acids Research,* 1986, vol. 14, 8135-8153
- **FRANK SEELA ; KLAUS KAISER.** *Nucleic Acids Research,* 1986, vol. 14, 1825-1844
- **J. F. HABENER et al.** *Proceedings of the National Acaddemy of Sciences,* 1988, vol. 85, 1735-1739
- **P. K. THOO et al.** *Nuclic Acids Research,* 1989, vol. 17, 10373-10383
- **D. E. BERGSTROM et al.** *Journal of American Chemical Society,* 1995, vol. 117, 1201-1209
- **D. LOAKES et al.** *Nucl. Acids Res.,* 1995, vol. 23, 2361-2366
- **D. LAOKES.** Survey and Summary: The applications of universal DNA base analogues. *Nucleic Acids Research,* 2001, vol. 29, 2437-2447
- **ZHANG et al.** Peptide Nucleic Acid-DNA Duplexes Containing the Universal Base 3-Nitropyrrole. *METHODS,* 2001, vol. 23, 132-140
- **CHALLA et al.** Nitroazole Universal Bases in Peptide Nucleic Acids. *Organic Letters,* 1999, vol. 1, 1639-1641
- **HEMAVATHI et al.** *Organic Letters,* 1999, vol. 1 (10), 1639-1641
- **KATHRYN A. F et al.** *Chemical Communincation,* 2002, 2206-2207
- **H. CHALLA et al.** *Organic Letters,* 1999, vol. 1, 1639

SEQUENCE LISTING

<110> Panagene Inc.

<120> Peptide nucleic acid oligomers comprising universal bases, preparation methods thereof, and kits, devices and methods for the analysis, detection or modulation of nucleic acids using the same

<130> 19623EP

<140> EP 07 851 574.9
<141> 2007-12-17

<150> KR-10-2006-0129517
<151> 2006-12-18

<160> 9

<170> KopatentIn 1.71

<210> 1
<211> 13
<212> DNA
<213> Artificial Sequence

<220>
<223> PNA oligomer

<400> 1
gagccatgag aaa 13

<210> 2

<211> 13
<212> DNA
<213> Artificial sequence

<220>
<223> PNA oligomer, n = 5-nitroindole

<400> 2
gagccatgng aaa 13

<210> 3
<211> 14
<212> DNA
<213> Artificial Sequence

<220>
<223> PNA oligomer, n = 5-nitroindole

<400> 3
gagccatgng aaac 14

<210> 4
<211> 14
<212> DNA
<213> Artificial Sequence

<220>
<223> PNA oligomer, n = 5-nitroindole

<400> 4
gagccatgag naac 14

<210> 5
<211> 13
<212> DNA
<213> Artificial Sequence

<220>
<223> PNA oligomer, n = 3-nitropyrrole

<400> 5
gagccatgng aaa 13

<210> 6
<211> 14
<212> DNA
<213> Artificial Sequence

<220>
<223> PNA oligomer, n = 3-nitropyrrole

<400> 6
gagccatgng aaac 14

<210> 7
<211> 14
<212> DNA
<213> Artificial Sequence

<220>
<223> PNA oligomer, n = 3-nitropyrrole

<400> 7
gagccatgag naac 14

<210> 8
<211> 20
<212> DNA
<213> Artificial Sequence

<220>
<223> Hbv-F primer

<400> 8
ccatcatctt gggctttcgc 20

<210> 9
<211> 25
<212> DNA
<213> Artificial Sequence

<220>
<223> Hbv-R primer

<400> 9
caaaagaaaa ttggtaacag cggta 25